# EUROPÄISCHE PATENTSCHRIFT

(11) **EP 1 163 886 B1**
(45) Veröffentlichungstag und Bekanntmachung des Hinweises auf die Patenterteilung: **10.05.2006**
(21) Anmeldenummer: 01113968.0
(22) Anmeldetag: 08.06.2001
(51) Int. Cl.: A61B 18/14, A61B 18/16

(54) **HF-resektoskopisches Instrument**
HF resectoscope
Résectoscope HF

(30) Priorität: 16.06.2000 DE 10028850
(43) Veröffentlichungstag der Anmeldung: 19.12.2001
(73) Patentinhaber: OLYMPUS WINTER & IBE GMBH, 22045 Hamburg (DE)
(72) Erfinder: Brommersma, Pieter, 22941 Bargteheide (DE); Kikuchi, Yasuhiko, Sagamihara, Kanagawa (JP)
(74) Vertreter: Schaefer, Konrad

(56) Entgegenhaltungen:
- US-A- 4 116 198
- US-A- 5 196 011
- US-A- 5 919 191
- US-A- 5 993 445

## Beschreibung

Die Erfindung betrifft ein HF-resektoskopisches Instrument zum Schneiden von Körpergewebe in einem mit leitfähiger Flüssigkeit gefüllten Körperhohlraum, z.B. der menschlichen Blase, gemäß dem Oberbegriff des Anspruchs 1. Derartige Instrumente werden zu ihrer bestimmungsgemäßen Verwendung typischerweise in ein Resektoskop eingebaut.

Die klassische Resektoskopie bedient sich der unipolaren bzw. monopolaren Technik. Dabei fließt ein HF-Strom von der Resektionselektrode, der aktiven Elektrode, durch den Körper des Patienten zu einer großflächigen Neutralelektrode, die außen am Patienten, beispielsweise an dessen Oberschenkel, angeordnet ist.

Mit dem Stromfluß durch den Körper des Patienten sind aber gewisse Risiken verbunden, die selbst bei sachgemäßer Handhabung des Resektoskopes niemals gänzlich auszuschalten sind. Zu nennen ist in diesem Zusammenhang insbesondere das Auftreten von unkontrollierbaren Leckströmen bzw. vagabundierenden Strömen, die z.B. bei Kontakt des Patienten mit einem metallischen Gegenstand, wie etwa dem Operationstisch, zu schmerzhaften Hautverbrennungen führen können. Weiterhin besteht bei strominduzierten Muskelkontraktionen die Gefahr, daß der Patient unkontrollierte, plötzliche Bewegungen ausführt, die zu Schnittverletzungen durch das resektoskopische Instrument führen können. Es besteht zudem latent die Gefahr, daß Muskel oder Nerven in der Umgebung des Resektionsgebietes zumindest zeitweise durch vagabundierende Ströme geschädigt werden.

Die oben genannten Risiken lassen sich bei Anwendung bipolarer Techniken weitestgehend ausschalten. Diesen ist gemein, daß nicht nur die Aktivelektrode, sondern auch die Neutralelektrode in den Körper des Patienten eingebracht wird, so daß der HF-Strom nur zwischen den beiden Elektroden des Instruments fließt, nicht aber, oder nur über definierte kurze Strecken, durch den Körper des Patienten.

Ein solches gattungsgemäßes HF-resektoskopisches Instrument zeigen die DE-OS-25 21 719 und die inhaltsgleiche, die Priorität der genannten DE-Schrift beanspruchende US 4,116,198, die als nächstliegender Stand der Technik angesehen werden. Dort ist die Neutralelektrode am Schlingenträgerarm angeordnet und kann mit diesem in den Schaft eines Resektoskopes ein- und ausgefahren werden. Das hat den Vorteil, daß der Abstand zwischen Aktiv- und Neutralelektrode gleichbleibend ist, und damit weitgehend gleichbleibende Stromwege gegeben sind, so daß die Schneidwirkung der Schneidelektrode bei jeder ausgefahrenen Stellung des Instrumentes unabhängig vom Grad des Ausfahrens näherungsweise konstant ist.

Bei diesen gattungsgemäßen Instrumenten befindet sich die Neutralelektrode in der Flüssigkeit in Abstand zum Körpergewebe. Bei Gewebekontakt der Aktivelektrode fließt HF-Strom eine kurze Strecke durch das Körpergewebe, tritt dann in die Flüssigkeit aus und gelangt durch die Flüssigkeit zurück zur Neutralelektrode.

Probleme bei der bipolaren Technik bereiten allerdings die Stromverluste, die durch den direkten Stromfluß durch die gut leitende Flüssigkeit zwischen Aktiv- und Neutralelektrode ohne Durchgang durch das Körpergewebe zustande kommen. In der Konsequenz führt deshalb nur ein Teil des in das HF-Instrument eingespeisten Stromes zu einer Schneidwirkung der Aktivelektrode, namentlich der Anteil, der von der Aktivelektrode in das Körpergewebe übergeht, und von dort zur Neutralelektrode zurückfließt.

Diese Stromverluste treten verstärkt insbesondere dann auf, wenn in Körperhohlräumen gearbeitet wird, in denen sich eine besonders gut leitende Flüssigkeit befindet, z.B. wenn während der Resektion mit einer elektrolytreichen z.B. isotonischen Spülflüssigkeit gespült wird. In diesen Fällen wird sogar der überwiegende Teil des Stromes direkt von der Schneid- bzw. Aktivelektrode zur Neutralelektrode fließen, ohne Schneidwirkung zu entfalten. Ein Schneiden ist deshalb mit aus dem Stand der Technik bekannten HF-resektoskopischen Instrumenten in mit leitfähiger Flüssigkeit gefüllten Körperhohlräumen nicht oder nicht zufriedenstellend möglich.

Diesem Problem wird im Stand der Technik dadurch begegnet, daß beim Spülen in der Regel schlecht leitende Spülflüssigkeiten eingesetzt werden. Wenn man sich aber vor Augen führt, daß bei der Resektion Blutgefäße eröffnet werden, so daß ein Teil der Flüssigkeit unvermeidlich in den Blutkreislauf des Patienten gelangt, und daß dies sich in einem Komplex von Symptomen, auch als TUR-Syndrom bekannt, äußern kann, wie Erbrechen, Herzrhythmusstörungen, Nierenversagen, Schock, so erscheint dies als eine ungeeignete Lösung, zumal die Symptome bei der Verwendung von isotonischen Flüssigkeiten in geringerem Maße bis gar nicht auftreten.

Es ist deshalb die Aufgabe der vorliegenden Erfindung ein HF-resektoskopisches Instrument zur Verfügung zu stellen, das die aufgezeigten Probleme überwindet, und damit für die Resektion von Körpergewebe in einem mit gut leitfähiger Flüssigkeit gefüllten Körperhohlraum optimiert ist.

Diese Aufgabe wird mit den Merkmalen des Anspruchs 1 gelöst.

Die Wirkung und damit der Vorteil der erfindungsgemäßen Anordnung eines Isolatorkörpers zwischen Schneid- und Neutralelektrode besteht darin, daß der direkte Stromfluß zwischen den beiden Elektroden erschwert und mithin vermindert wird, wodurch ein größerer Anteil der in das HF-Instrument eingespeisten Stromleistung von der Schneidelektrode in das Körpergewebe übergeht und dort Schneidwirkung entfaltet. Umgekehrt betrachtet bedeutet dies, daß bei dem erfindungsgemäßen Instrument zur Erzielung des gleichen Schneiderfolges gegenüber den bekannten Instrumenten weniger Leistung eingespeist werden muß.

Die prinzipielle Idee, einen Isolatorkörper zwischen der Aktivelektrode und der Neutralelektrode anzuordnen, ist aus der nicht gattungsgemäßen WO 97/24993 bekannt, die in Figur 9 ein Instrument mit einer rollbaren kugelförmigen Vaporationselektrode zeigt, die drehbar an einem Träger angebracht ist. Die Drehachse der Kugel steht dabei senkrecht zur Längsachse des Trägers. In geringem Abstand oberhalb der Kugel ist ein hemisphärisch gekrümmter Isolatorkörper, ebenfalls vom Träger gehalten, angeordnet, auf dessen der Vaporationselektrode abgewandten Seite die Neutralelektrode angeordnet ist. Es wird dadurch ein verringerter direkter Stromfluß zwischen Aktiv- und Neutralelektrode erreicht. Mit dieser Konstruktion des Isolatorkörpers wird dem Operateur jedoch ein Großteil des Sichtfeldes verdeckt. Dieser Nachteil ist bei der verwendeten Kugelelektrode unvermeidbar.

Die Neutralelektrode des bipolaren HF-Instrumentes ließe sich prinzipiell an irgendeiner beliebigen Stelle des Schlingenträgers anordnen. Es ist allerdings gemäß Anspruch 2 bevorzugt, daß die Neutralelektrode distal am Schlingenträger angeordnet ist. Dies hat den Vorteil, daß die Neutralelektrode beim Einfahren des Instrumentes in das Schaftrohr des Resektoskopes nicht an der im Schaftrohr angeordneten Beobachtungs- bzw. Beleuchtungsoptik vorbeigeführt werden muß, sondern bei geeigneter Wahl seiner Abmessungen im eingefahrenen Zustand distal vor der Optik liegt. Es ist dabei zur Vermeidung elektrischer Überschläge dafür Sorge zu tragen, daß eine Berührung von Neutralelektrode und Optik unterbleibt. Es versteht sich, daß dabei der Isolatorkörper ebenfalls distal am Schlingenträger anzuordnen ist, um seine abschirmende Funktion in Anordnung zwischen den Elektroden erfüllen zu können.

Die Schneidelektrode, der Isolatorkörper und die Neutralelektrode sind allesamt am Schlingenträger angeordnet und werden von ihm gehalten. In der allgemeinsten Form können die drei unabhängig voneinander am Schlingenträger gehalten sein, z.B. jeweils mittels vom Schlingenträger abgehender Haltearme. Dies ist aber nicht nur in der Herstellung aufwendig, sondern auch von geringer Stabilität. Es ist deshalb gemäß Anspruch 3 vorteilhaft, daß die Neutralelektrode direkt auf der der Schneidelektrode abgewandten Seite des Isolatorkörpers angebracht ist. Der Isolatorkörper trägt somit die Neutralelektrode und gibt ihr Stabilität. Ferner wird dadurch die Abschirmgeometrie verbessert.

Die Form der Neutralelektrode ist weitgehend beliebig. Es ist aber nach Anspruch 4 bevorzugt, daß die Neutralelektrode flächig auf dem Isolatorkörper angebracht ist, wodurch sie einen besseren Halt erhält. Weiterhin hat die flächige Ausbildung der Neutralelektrode den Vorteil, daß der von der Schneidelektrode ins Gewebe fließende und von dort zur Neutralelektrode zurückfließende Strom großflächig und damit mit geringer Stromdichte in die Neutralelektrode eintreten kann. Umgekehrt bedeutet dies aber auch, daß bei versehentlichem Kontakt der flächigen Neutralelektrode mit Körpergewebe nur eine geringe Wirkung auf dasselbe entsteht. Zusätzlich wird die Abschirmgeometrie weiter verbessert.

Die Stabilität der Anordnung der Neutralelektrode und die Abschirmgeometrie läßt sich dadurch weiter verbessern, daß die Neutralelektrode gemäß Anspruch 5 in den Isolatorkörper eingelassen ist. Es ist weiterhin damit sichergestellt, daß die Neutralelektrode im in das Resektoskop eingefahrenen Zustand nicht in leitenden Kontakt mit dem Schaft oder der Optik gelangen kann.

Wenn die Neutralelektrode allerdings vollständig in den Isolatorkörper eingelassen ist, ergibt sich das Problem, daß bei Erreichen der Schneidkante des Resektoskopes durch die Schneidelektrode die Neutralelektrode zum einen vom Isolatorkörper und zum anderen vom in der Regel isoliert ausgebildeten distalen Endbereich des Resektoskopschaftes verdeckt wird. Die Folge ist eine Unterbrechung des Stromflusses, so daß die Schneidwirkung abbricht, bevor der abgeschnittene Gewebestreifen an der Schneidkante abgetrennt worden ist.

Dies läßt sich in einfacher und vorteilhafter Weise gemäß Anspruch 6 dadurch vermeiden, daß die Neutralelektrode einen distal nicht von dem Isolatorkörper abgedeckten Bereich aufweist. Dieser Bereich ist auch im eingefahrenen Zustand bzw. bei Erreichen der Schneidkante des Resektoskopes durch die Schneidelektrode unverdeckt und damit für den rückfließenden Strom frei zugänglich, so daß weiterhin der Stromfluß gewährleistet ist, und der Körpergewebestreifen an der Schneidkante abgetrennt werden kann.

Der Isolatorkörper kann z.B. als ebene Platte ausgebildet sein. Es ist allerdings gemäß Anspruch 7 vorteilhaft, daß der Isolatorkörper entgegengesetzt zur Schneidelektrode gekrümmt ausgebildet ist. Mit der Krümmung wird die Sicht des Operateurs durch den Isolatorkörper und die Schneidelektrode hindurch in vorteilhafter Weise verbessert und die Elektrode kann beim Einfahren zudem einfacher über die Optik des Resektoskopes geführt werden, insbesondere bei einer exzentrisch oberhalb der Mittelachse des Resektoskops angeordneten Optik.

Der die Schneidelektrode haltende Schlingenträger kann z.B. einarmig ausgebildet sein. In einem solchen Fall spricht man auch von Einstrang-Elektroden. Üblicherweise gabelt sich der Schlingenträger aber zum distalen Ende hin in zwei parallele Stangen, zwischen denen geneigt zur Längsachse des Schlingenträgers im wesentlichen halbkreisförmig die auch als Schneidschlinge bezeichnete Schneidelektrode gehalten ist. Die Schlingenebene steht dabei in der Regel annähernd senkrecht zur Längsachse des Schlingenträgers. Die Erfindung ist aber darauf nicht beschränkt, sondern umfaßt auch von 90° abweichende Neigungswinkel.

Für den Fall gegabelter Schlingenträger wird mit Vorteil vorgeschlagen, daß der Isolatorkörper an beiden Stangen distal ansetzt. Der Isolatorkörper sorgt dadurch für eine gute mechanische Stabilität des distalen Bereiches des HF-resektoskopischen Instrumentes. Bevorzugt ist der Isolatorkörper auch bei gegabelten Schlingenträgern entgegengesetzt zur von den Stangen getragenen Schneidelektrode gekrümmt. Auf diese Weise verbleibt zwischen Schneidelektrode und Isolatorkörper ein freier zentraler Bereich, durch den eine ungestörte Beobachtung des Operationsfeldes durch den Operateur möglich ist.

Weiterhin ist es nach Anspruch 9 vorteilhaft, daß die Achsprojektion der Schneidelektrode zusammen mit der des gekrümmten Isolators mit der darauf angeordneten Neutralelektrode im wesentlichen einen Kreis bildet. Es versteht sich, daß der Durchmesser dieses Kreises geringfügig kleiner als der Innendurchmesser des Schaftrohres des Resektoskopes zu sein hat. Es versteht sich ebenfalls, daß bei Resektoskopen mit ovalem Querschnitt des Schaftrohres auch die oben genannte Achsprojektion ovalen Charakter erhalten sollte. Die Vorteile entsprechen den im vorherigen Absatz genannten, nämlich insbesondere eine freie Sicht auf das Operationsfeld.

Es ist nach Anspruch 10 vorteilhaft, daß der Isolatorkörper einstückig aus einem entgegengesetzt zur Schneidelektrode gekrümmten Teil und einem dem Verlauf der Schneidelektrode folgenden distal zu dieser angeordneten Teil besteht, so daß der Isolatorkörper für sich genommen bereits im wesentlichen einen Ring ausbildet. Dies trägt insbesondere in Verbindung mit der nach Anspruch 11 vorgeschlagenen Befestigung der Schneidelektrode an dem ihr folgenden Teil des Isolatorkörpers zu einer sehr stabilen Konstruktion bei. Die erfindungsgemäßen Elektroden besitzen eine lange Lebensdauer, da der bei Gebrauch des HF-Instrumentes besonders beanspruchte Schneidschlingenbereich durch den unteren Isolatorkörperteil mechanisch stabilisiert wird. Es ergeben sich weiterhin Vorteile bei der Montage des Instrumentes und eine günstige Abschirmung der Schneidschlinge durch den Isolatorkörper.

Es ist nach Anspruch 12 bevorzugt, daß die Schneidelektrode als im unteren Bereich zu einer Platte verbreiterter Draht ausgebildet ist, wobei die Platte auf der Außenfläche des dem Verlauf der Schneidelektrode folgenden Teils des Isolatorkörpers angeordnet ist. Bei dieser mechanisch stabilen Anordnung kann die Unterseite des plattenförmigen Bereiches eine Koagulation bewirken, während die Oberseite durch den Isolatorkörper abgeschirmt ist. Indem also erfindungsgemäß die Bereiche der Schneidelektrode durch den Isolatorkörper abgeschirmt werden, die nicht in Gewebekontakt stehen bzw. stehen sollen, wird erreicht, daß der direkte Stromfluß zwischen Schneid- und Neutralelektrode effektiv weiter verringert wird.

In den nachfolgenden Zeichnungen, in denen die Erfindung an Hand von Ausführungsbeispielen schematisch dargestellt ist, sind weitere Einzelheiten und Merkmale der Erfindung ersichtlich. Es zeigen:
- Figur 1: eine Perspektivansicht des distalen Endbereiches eines eifindungsgemäßen HF-resektoskopischen Instrumentes mit einer Drahtschlinge als Aktivelektrode,
- Figur 2: eine Ansicht gemäß Figur 1 des Instrumentes in einer Variante mit einem Band als Aktivelektrode,
- Figur 3: eine Ansicht gemäß Figur 2 mit einem alternativen Isolatorkörper und einer Drahtschlinge mit zu einer Platte erweitertem unteren Bereich als Aktivelektrode,
- Figur 4: eine Seitenansicht des Instrumentes gemäß Figur 1 im aus dem Schaftrohr eines längsgeschnitten dargestellten Resektoskopes ausgefahrenen Zustand,
- Figur 5: eine Seitenansicht wie Figur 4 im eingefahrenen Zustand, und
- Figur 6: eine Frontansicht zu Figur 5.

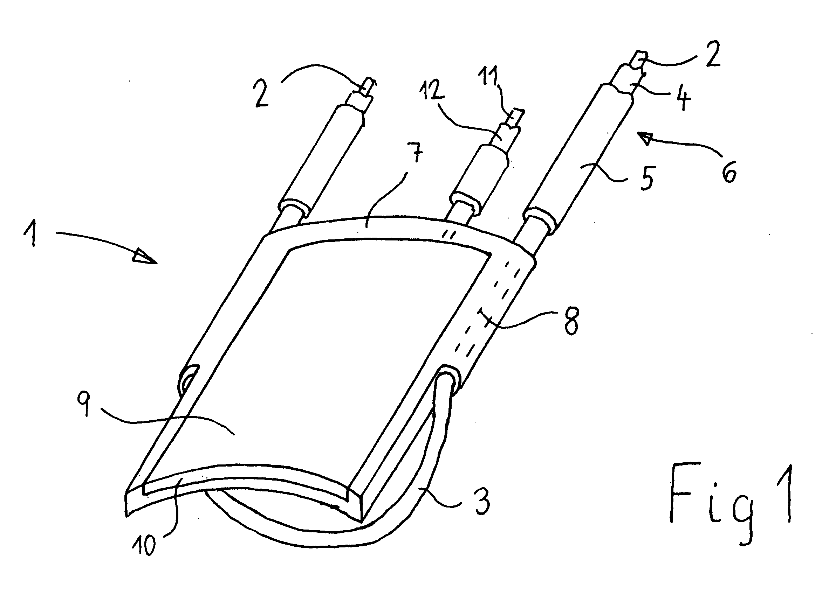

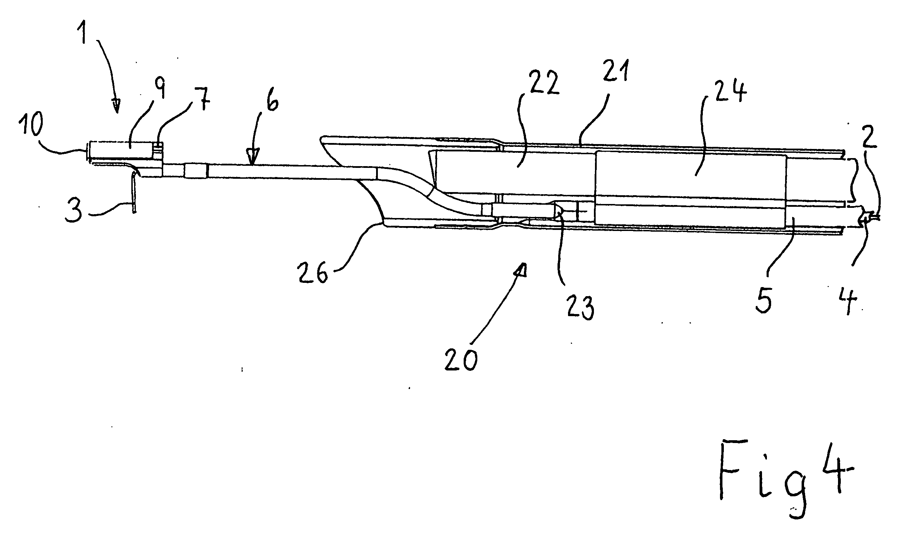

Figur 1 zeigt den distalen Endbereich eines erfindungsgemäßen HF-resektoskopischen Instrumentes 1, das zu seinem bestimmungsgemäßen Gebrauch wie die Figuren 4 bis 6 zeigen in einem Resektoskop auswechselbar angeordnet befestigt und von einem nicht gezeigten HF-Generator mit Strom beaufschlagt wird.

Das Instrument 1 ist wie in Figur 1 und 4 dargestellt in seiner gesamten Länge von einem Innenleiter 2 durchzogen, der mit Ausnahme seines proximalen Kontaktbereiches und seines distal als Schneidschlinge 3 ausgebildeten Bereichs von einer Isolatorhülle 4 umgeben ist, die wiederum innerhalb eines aus einem formstabilen Material hergestellten Rohres 5 verläuft. Zusätzlich kann der distale Bereich zur Erhöhung der mechanischen Stabilität und der elektrischen Sicherheit z.B. mit einer Keramik-Verstärkung ausgerüstet sein. Zum distalen Ende hin gabelt sich das Instrument 1 in zwei weitgehend parallel zueinander verlaufende Schlingenträgerarme 6, wobei Fig. 1 nur den distalen Abschnitt nach der Gabelung darstellt.

Zwischen den Schlingenträgerarmen 6 und von diesen gehalten erstreckt sich ein nach oben zylindrisch gebogener, flächig ausgebildeter Isolatorkörper 7, durch den mittels in dessen Flanken ausgebildeter Bohrungen 8 der Innenleiter 2 in axialer Richtung bis etwa zur halben Länge des Isolatorkörpers 7 verläuft, um dort auszutreten und in einem nach unten gekrümmten Bogen die Schneidelektrode in Form einer Schneidschlinge 3 zu bilden, wobei die Schlingenebene der Schneidschlinge 3 in etwa senkrecht zur Längsachse des Instrumentes 1 steht. Die Schlinge 3 befindet sich demzufolge etwa mittig unterhalb des Isolatorkörpers 7, auf dessen Oberseite eine flächige und in gleicher Weise wie der Isolatorkörper 7 zylindrisch gekrümmte Neutralelektrode 9 eingelassen ist, die z.B. eine Metallplatte sein kann.

Während der Isolatorkörper 7 zum proximalen Ende hin die Neutralelektrode 9 abschirmt, weist die Neutralelektrode 9 am distalen Ende einen vom Isolatorkörper 7 unverdeckten, d.h. freiliegenden Bereich 10 auf. Über eine seitlich verlaufende Leitung 11, die von einer Isolatorhülle 12 umgeben ist, und die z.B. vorteilhaft durch eines der Gabelrohre isoliert vom Innenleiter 2 geführt sein kann, steht die Neutralelektrode 7 mit einem Pol des nicht dargestellten HF-Generators in leitender Verbindung, während die Schneidschlinge 3 über den Innenleiter 2 an dessen anderen Pol angeschlossen ist.

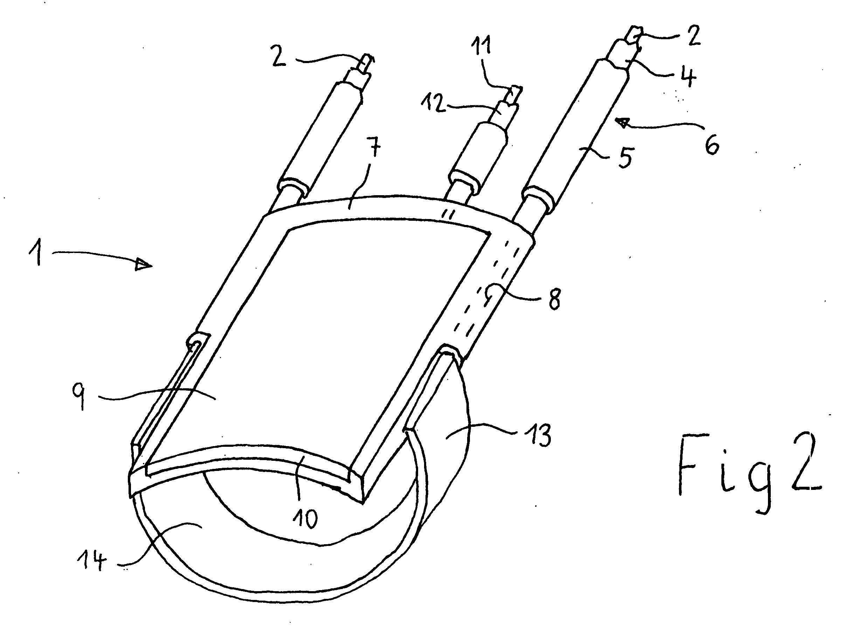

Figur 2 zeigt eine Ausführungsvariante des HF-Instrumentes 1, die der in Fig. 1 gezeigten weitgehend entspricht, wobei die Schneidelektrode hier von einer Bandelektrode 13 gebildet wird, die auf ihrer innenliegenden Fläche zusätzlich mit einer Isolierschicht 14 versehen sein kann. Es werden ansonsten bei den übereinstimmenden Teilen dieselben Bezugszeichen wie in Figur 1 verwendet.

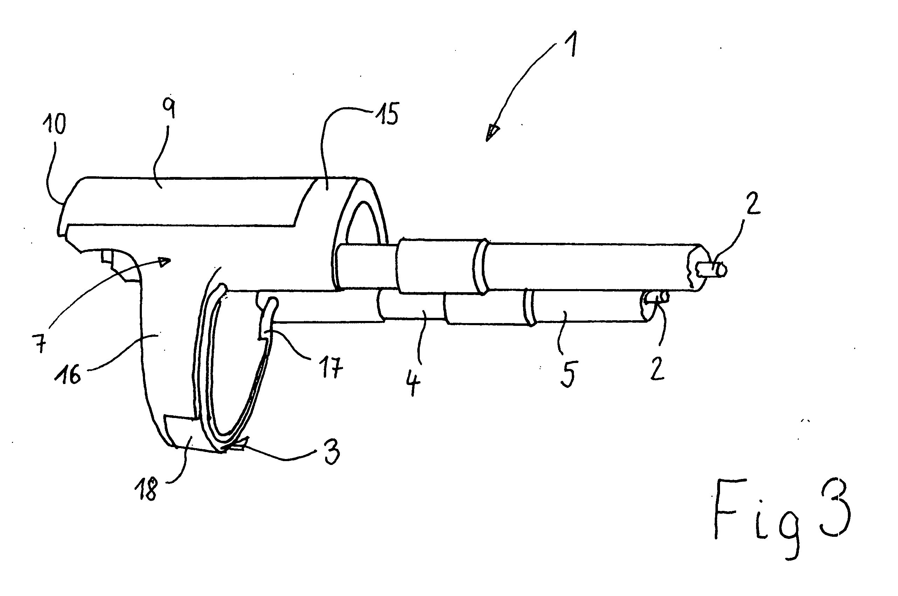

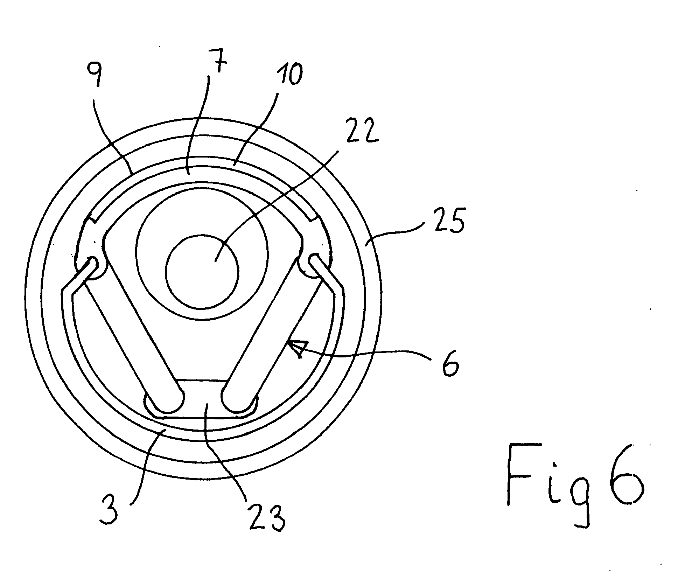

Eine vorteilhafte alternative Ausführungsform des Isolatorkörpers 7 zeigt Figur 3. Zusätzlich zu dem zylindrisch nach oben gekrümmten oberen Teil 15 weist der Isolatorkörper 7 ein gegensinnig dazu nach unten gekrümmtes unteres Teil 16 auf, wobei der Isolatorkörper 7 einstückig hergestellt ist, z.B. aus einer Keramik. In Achsprojektion des Isolatorkörpers 7 bildet dieser im wesentlichen einen Ring aus. Für den Isolatorkörper 7 aus den Figuren 1 und 2 ist in Figur 6 zu erkennen, daß dieser zusammen mit der Schneidschlinge 3 ebenfalls in Achsprojektion einen Ring ausbildet.

Die Statik des distalen Bereiches der HF-Elektrode 1 wird im wesentlichen von dem einstückigen Isolatorkörper 7 geprägt, an dem die in ihrem oberen Bereich 17 drahtförmig und im unteren Bereich flächig in Form einer gekrümmten Platte 18 ausgebildete Schneidelektrode 3 befestigt ist. Dadurch erhält auch die Schneidelektrode 3 eine gute Stabilität.

Neben diesen drei beispielhaft gezeigten Formen der Schneidelektrode sind auch weitere im Stand der Technik bekannte Ausbildungen möglich, z.B. eine mit Rillen oder Profil versehene Schneidelektrode. Die Erfindung ist nicht auf eine spezielle Ausführungsform der Schneidelektrode beschränkt.

Beim Einsatz des HF-Instrumentes 1 zum Schneiden von Körpergewebe in einem mit leitfähiger Flüssigkeit gefüllten Körperhohlraum befindet sich nur die Schneidelektrode 3 in leitendem Kontakt mit dem Gewebe. An dieser Kontaktstelle tritt der Strom kleinflächig, d.h. mit hoher Stromdichte, in das zu entfernende Gewebe ein, und entfaltet dadurch seine Schneidwirkung. Der Schneidstrom durchläuft eine im Mittel nur kurze Strecke durch das Gewebe und fließt dann, großflächig aus dem Gewebe austretend, durch die leitende Flüssigkeit zur auf der Oberseite des Isolatorkörpers 7 angebrachten Neutralelektrode 9 zurück. Zusätzlich zu diesem Nutzstrom fließt ein Verluststrom ohne Schneidwirkung zu entfalten durch die Flüssigkeit direkt von der Schneidelektrode 3 zur Neutralelektrode 9, wobei dieser Anteil dadurch niedrig gehalten wird, daß der Isolatorkörper 7 die geradlinigen Verbindungswege zwischen den beiden Elektroden 3 und 9 abschirmt, den Strom also zwingt, einen längeren Weg zurückzulegen und dadurch quasi den Widerstand in der Flüssigkeit relativ zum Widerstand des Stromflusses durch das Körpergewebe erhöht. Da der Strom aber dem Weg des geringsten Widerstandes bevorzugt folgt, bewirkt die Anordnung des Isolatorkörpers 7 einen erhöhten Stromfluß durch das Körpergewebe, bzw. eine Verringerung des Verluststromes.

Bei Figur 3 ist zu erkennen, daß durch das untere Teil 16 des Isolatorkörpers 7 zusätzlich eine Abschirmung des drahtförmigen Bereiches 17 der Schneidschlinge 3 zur proximalen Seite hin und des als Platte 18 ausgebildeten Bereiches der Schneidschlinge 3 nach oben hin erfolgt.

Figur 4 zeigt ein in einem handelsüblichen Resektoskop 20 eingebautes, erfindungsgemäßes Instrument 1 in der Ausführungsform der Figur 1 im ausgefahrenen Zustand, wobei die Vor- und Rückbewegung des Instrumentes in axialer Richtung über einen nicht gezeigten Transporteur des Resektoskopes erfolgt, mit dem das Instrument 1 verbunden ist. Das Resektoskop 20 besteht aus einem Schaftrohr 21, in dem die hier als exzentrisch angeordnet dargestellte Optik 22, an die sich proximal z.B. ein Okular oder ein Bildaufnahmegerät anschließen kann, und das HF-Instrument 1 aufgenommen sind. Weiterhin ist das Resektoskop 20 derart ausgestaltet, daß Spülflüssigkeit kontinuierlich aus dem Schaftrohr 21 austreten und auch wieder abgeführt werden kann, z.B. indem der Schaft als Doppelschaft mit Eintrittsöffnungen für den Rückfluß in seiner äußeren Wand ausgebildet ist. Die zugehörigen Zufluß- und Rückflußanschlüsse befinden sich dabei im nicht mehr gezeigten proximalen Bereich des Resektoskopes 20.

Proximal von der Gabelungsstelle 23 ist an dem Rohr 5 des Instrumentes 1 eine Führungshülse 24 befestigt, die im eingebauten Zustand die Optik 22 mit geringem Spiel und auf ihr gleitbar umschließt, so daß das Instrument 1 bei axialer Bewegung relativ zur Optik 22 stabilisierend gehalten ist. Im eingebauten Zustand liegt die Gabelungsstelle 23 unterhalb der Optik, jedoch erstrecken sich die beiden Schlingenträger 6 distal von der Gabelungsstelle 23 nach schräg oben, um seitlich an der Optik 22 vorbeigeführt zu werden.

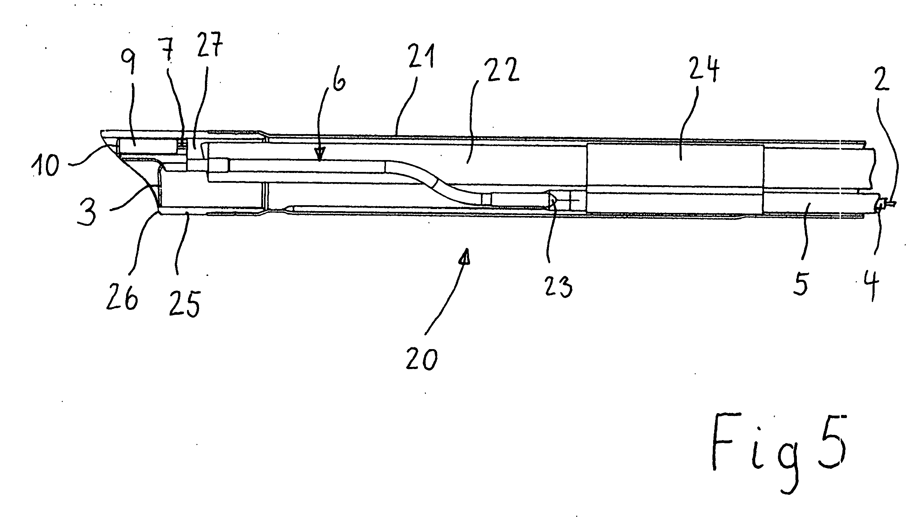

Dies ist in Figur 5 besser zu erkennen, in der das HF-Instrument 1 im eingefahrenen Zustand gezeigt ist, sowie in der zugehörigen Frontansicht der Figur 6. Die Schlingenträgerarme 6 verlaufen seitlich an der Optik 22 vorbei. Die Abmessungen des Instrumentes 1 sind so gewählt, daß es vollständig in das Resektoskop 20 einziehbar ist. Darunter soll verstanden sein, daß der distale Endbereich des HF-Instrumentes 1 im Seitenprofil vollständig vom distalen Schaftbereich des Resektoskopes 20 abgedeckt ist, der als schnabelförmige isolierte Spitze 25 dargestellt ist. Es ist zu erkennen, daß die Schneidschlinge 3 zumindest bis auf Höhe der Schneidkante 26 einziehbar ist, um einen Körpergewebestreifen rückziehend abschneiden zu können. Zwischen der Neutralelektrode 9 und der Optik 22 verbleibt ein freier Spalt 27. Bis auf den distal von dem Isolatorkörper 7 freigelassenen Bereich 10 wird die Neutralelektrode von der Resektoskopspitze 25 verdeckt.

In Figur 6 ist in einer Frontalansicht die einen kreisförmigen Querschnitt aufweisende Spitze 25 des Resektoskopes 20 zu erkennen. Seitlich oberhalb der üblicherweise exzentrisch oberhalb der Mittelachse angeordneten Optik 22, die z.B. als 12°-Optik ausgebildet ist, tragen die Schlingenträgerarme 6 die Schneidschlinge 3, die im wesentlichen halbkreisförmig und in Abstand zur Wandung der Spitze 25 nach unten gebogen verläuft. Der Isolatorkörper 7 setzt an den Schlingenträgerarmen 6 an und verläuft im Bogen oberhalb der Optik. Es ist hier zu sehen, daß zwischen der nach unten gebogenen Schneidschlinge 3 und dem nach oben gebogenen Isolatorkörper 7 bzw. der darauf angeordneten Neutralelektrode 9 ein etwa kreisrunder freier Raumbereich verbleibt, durch den der Operateur über die sich innerhalb dieses Fensters angeordnete Optik 22 freie Sicht auf das Operationsfeld hat. Auch eventuell über die Optik zugeführtes Beleuchtungslicht wird weder von der Schneidelektrode 3, noch von der Neutralelektrode 9 und dem Isolatorkörper 7 behindert.

In den Zeichnungen sind lediglich gegabelte Ausführungsformen dargestellt, ohne daß dadurch Einstrang-Elektroden ausgeklammert sein sollen. Dies gilt in gleicher Weise für die stets exzentrisch oberhalb der Schaftquerschnittmitte angeordnet dargestellte Optik, die natürlich auch mittig oder unterhalb der Schaftquerschnittmitte angeordnet sein kann, ohne die Erfindung zu verlassen.

## Patentansprüche

1. Zur Verwendung mit einem Resektoskop (20) bestimmtes HF-resektoskopisches Instrument (1) zum Schneiden von Körpergewebe in einem mit leitfähiger Flüssigkeit gefüllten Körperhohlraum, mit einem Schlingenträger (6) und einer distal daran angeordneten Schneidelektrode (3) in Form einer Schlinge mit einer zur Längsachse des Schlingenträgers (6) geneigten Schlingenebene, und mit einer am Schlingenträger (6) angeordneten Neutralelektrode (9), **dadurch gekennzeichnet, daß** am Schlingenträger (6) zwischen den Elektroden (3, 9) ein Isolatorkörper (7) derart angeordnet ist, daß jede gerade Verbindungslinie zwischen den Elektroden (3, 9) durch den Isolatorkörper (7) verläuft, wobei die Neutralelektrode (9) und der Isolatorkörper (7) in Arbeitsstellung im Resektoskop (20) mit in dessen Schaftrohr (21) angeordneter Optik (22) dessen Sicht weitgehend nicht behindernd angeordnet und/oder ausgebildet sind.

2. HF-resektoskopisches Instrument (1) nach Anspruch 1, **dadurch gekennzeichnet, daß** die Neutralelektrode (9) distal am Schlingenträger (6) angeordnet ist.

3. HF-resektoskopisches Instrument (1) nach Anspruch 1, **dadurch gekennzeichnet, daß** die Neutralelektrode (9) auf der der Schneidelektrode (3) abgewandten Seite des Isolatorkörpers (7) angebracht ist.

4. HF-resektoskopisches Instrument (1) nach Anspruch 3, **dadurch gekennzeichnet, daß** die Neutralelektrode (9) flächig auf dem Isolatorkörper (7) angebracht ist.

5. HF-resektoskopisches Instrument (1) nach Anspruch 3, **dadurch gekennzeichnet, daß** die Neutralelektrode (9) in den Isolatorkörper (7) eingelassen ist.

6. HF-resektoskopisches Instrument (1) nach Anspruch 5, **dadurch gekennzeichnet, daß** die Neutralelektrode (9) einen distal nicht von dem Isolatorkörper (7) abgedeckten Bereich (10) aufweist.

7. HF-resektoskopisches Instrument (1) nach Anspruch 1, **dadurch gekennzeichnet, daß** der Isolatorkörper (7) entgegengesetzt zur Schneidelektrode (3) gekrümmt ist.

8. HF-resektoskopisches Instrument (1) nach Anspruch 1, wobei sich der Schlingenträger (6) in zwei parallele Stangen gabelt, zwischen denen quer zur Achse im wesentlichen halbkreisförmig die Schneidelektrode (3) gehalten ist, **dadurch gekennzeichnet, daß** der Isolatorkörper (7) an beiden Stangen ansetzt.

9. HF-resektoskopisches Instrument (1) nach Anspruch 7, **dadurch gekennzeichnet, daß** die Achsprojektion der Schneidelektrode (3) zusammen mit der des gekrümmten Isolatorkörpers (7) mit der darauf angeordneten Neutralelektrode (9) im wesentlichen einen Kreis bildet.

10. HF-resektoskopisches Instrument (1) nach Anspruch 9, **dadurch gekennzeichnet, daß** der Isolatorkörper (7) einstückig aus einem entgegengesetzt zur Schneidelektrode (3) gekrümmten Teil (15) und einem dem Verlauf der Schneidelektrode (3) folgenden distal zu dieser angeordneten Teil (16) besteht.

11. HF-resektoskopisches Instrument (1) nach Anspruch 10, **dadurch gekennzeichnet, daß** die Schneidelektrode (3) an dem ihrem Verlauf folgenden Teil (16) des Isolatorkörpers (7) befestigt ist.

12. HF-resektoskopisches Instrument (1) nach Anspruch 11, **dadurch gekennzeichnet, daß** die Schneidelektrode (3) als im unteren Bereich zu einer Platte (18) verbreiterter Draht (17) ausgebildet ist, wobei die Platte (18) auf der Außenfläche des der Schneidelektrode (3) folgenden Teils (16) des Isolatorkörpers (7) angeordnet ist.

## Claims

1. HF resectoscopic instrument (1) intended for use with a resectoscope (20) for cutting body tissue in a body cavity filled with a conductive fluid, with a loop support (6) and a cutting electrode (3) in the form of a loop mounted distally thereon with a loop plane inclined relative to the longitudinal axis of the loop support (6), and with a neutral electrode (9) disposed on the loop support (6), **characterised in that** an insulator body (7) disposed on the loop support (6) between the electrodes (3, 9) in such a way that each straight connecting line between the electrodes (3, 9) extends through the insulator body (7), wherein in the working position the neutral electrode (9) and the insulator body (7) are disposed and/or constructed in the resectoscope (20) with an optical unit (22) disposed in the stem tube (21) thereof so that they do not significantly hinder a view through the optical unit.

2. HF resectoscopic instrument (1) as claimed in Claim 1, **characterised in that** the neutral electrode (9) is disposed distally on the loop support (6).

3. HF resectoscopic instrument (1) as claimed in Claim 1, **characterised in that** the neutral electrode (9) is mounted on the side of the insulator body (7) facing away from the cutting electrode (3).

4. HF resectoscopic instrument (1) as claimed in Claim 3, **characterised in that** the neutral electrode (9) is mounted flat on the insulator body (7).

5. HF resectoscopic instrument (1) as claimed in Claim 3, **characterised in that** the neutral electrode (9) is embedded in the insulator body (7).

6. HF resectoscopic instrument (1) as claimed in Claim 5, **characterised in that** the neutral electrode (9) has a region (10) which is not covered distally by the insulator body (7).

7. HF resectoscopic instrument (1) as claimed in Claim 1, **characterised in that** the insulator body (7) is curved in the opposite direction to the cutting electrode (3).

8. HF resectoscopic instrument (1) as claimed in Claim 1, wherein the loop support (6) is bifurcated into two parallel bars between which the cutting electrode (3) is retained in substantially semi-circular form transversely with respect to the axis, **characterised in that** the insulator body (7) adjoins both bars.

9. HF resectoscopic instrument (1) as claimed in Claim 7, **characterised in that** the axial projection of the cutting electrode (3) together with that of the curved insulator body (7) with the neutral electrode (9) disposed thereon basically forms a circle.

10. HF resectoscopic instrument (1) as claimed in Claim 9, **characterised in that** the insulator body (7) is integrally formed from a part (15) which is curved in the opposite direction to the cutting electrode (3) and a part (16) which distally follows the contour of the cutting electrode (3).

11. HF resectoscopic instrument (1) as claimed in Claim 10, **characterised in that** the cutting electrode (3) is fixed on the part (16) of the insulator body (7) which follows the contour thereof.

12. HF resectoscopic instrument (1) as claimed in Claim 11, **characterised in that** the cutting electrode (3) is constructed as a wire (17) which is widened to form a plate (18) in the lower region, wherein the plate (18) is disposed on the outer face of the part (16) of the insulator body (7) which follows the cutting electrode (3).

## Revendications

1. Instrument résectoscopique HF (1) destiné à être utilisé avec un résectoscope (20) pour sectionner des tissus corporels dans une cavité corporelle remplie d'un liquide conducteur, comportant un support d'anse (6) à la partie distale duquel est agencée une électrode de résection (3) en forme d'anse selon un plan incliné par rapport à l'axe longitudinal du support d'anse (6), et une électrode neutre (9) sur le support d'anse (6), **caractérisé en ce que** le support d'anse (6) porte, entre les électrodes (3, 9), un corps isolant (7) disposé de façon telle que toute ligne de jonction droite entre les électrodes (3, 9) passe par le corps isolant (7), l'électrode neutre (9) et le corps isolant (7) étant agencés et/ou conformés de façon telle que, lorsqu'ils se trouvent en leur position fonctionnelle dans le résectoscope (20) doté, dans sa gaine (21), d'une optique (22), ils ne gênent pas la vue de cette dernière.

2. Instrument résectoscopique HF (1) selon la revendication 1, **caractérisé en ce que** l'électrode neutre (9) est disposée à la partie distale du support d'anse (6).

3. Instrument résectoscopique HF (1) selon la revendication 1, **caractérisé en ce que** l'électrode neutre (9) est fixée sur la face,du corps isolant (7) opposée à l'électrode de résection (3).

4. Instrument résectoscopique HF (1) selon la revendication 3, **caractérisé en ce que** l'électrode neutre (9) est fixée par toute sa surface sur le corps isolant (7).

5. Instrument résectoscopique HF (1) selon la revendication 3, **caractérisé en ce que** l'électrode neutre (9) est encastrée dans le corps isolant (7).

6. Instrument résectoscopique HF (1) selon la revendication 5, **caractérisé en ce que** l'électrode neutre (9) présente dans sa partie distale une section (10) non masquée par le corps isolant (7).

7. Instrument résectoscopique HF (1) selon la revendication 1, **caractérisé en ce que** le corps isolant (7) est incurvé selon une courbure opposée à celle de l'électrode de résection (3).

8. Instrument résectoscopique HF (1) selon la revendication 1, le support d'anse (6) se ramifiant en deux tiges parallèles entre lesquelles est maintenue, transversalement par rapport à l'axe, l'électrode de résection (3) de forme essentiellement semi-circulaire, **caractérisé en ce que** le corps isolant (7) est fixé aux deux tiges.

9. Instrument résectoscopique HF (1) selon la revendication 7, **caractérisé en ce que** la projection axiale de l'électrode de résection (3) et celle du corps isolant (7) incurvé portant l'électrode neutre (9) disposée sur celui-ci forment ensemble une figure essentiellement circulaire.

10. Instrument résectoscopique HF (1) selon la revendication 9, **caractérisé en ce que** le corps isolant (7) est réalisé d'un seul tenant comportant une partie (15) incurvée selon une courbure opposée à celle de l'électrode de résection (3) et une partie (16) distale par rapport à l'électrode de résection (3) et qui suit l'incurvation de cette dernière.

11. Instrument résectoscopique HF (1) selon la revendication 10, **caractérisé en ce que** l'électrode de résection (3) est fixée à la partie (16) du corps isolant (7) qui en suit l'incurvation.

12. Instrument résectoscopique HF (1) selon la revendication 11 **caractérisé en ce que** l'électrode de résection (3) est réalisée sous forme de fil (17) qui, dans la partie inférieure, s'élargit pour former une plaque (18) disposée contre la face extérieure de la partie (16) du corps isolant (7) qui suit l'incurvation de l'électrode de résection (3).
